Europäisches Patentamt

European Patent Office

Office européen des brevets

(11) Publication number : **0 490 571 A2**

(12) # EUROPEAN PATENT APPLICATION

(21) Application number : 91311285.0

(51) Int. Cl.⁵ : **C07C 41/06,** C07C 43/04

(22) Date of filing : 04.12.91

(30) Priority : 13.12.90 GB 9027112

(43) Date of publication of application :
17.06.92 Bulletin 92/25

(84) Designated Contracting States :
AT BE CH DE DK ES FR GB GR IT LI LU NL SE

(71) Applicant : THE BRITISH PETROLEUM
COMPANY P.L.C.
Britannic House, 1 Finsbury Circus
London EC2M 9BA (GB)

(72) Inventor : Greenough, Paul
BP Engineering, Uxbridge One, 1 Harefield
Road
Uxbridge, Middlesex UB8 1PD (GB)
Inventor : Hochstadt, Gunter
Talstrasse 56
W-7899 Grenzach-Wyhlen 1 (DE)
Inventor : Langmack, Heinz
Suderstrasse 3
W-2200 Elmshorn (DE)

(74) Representative : Crack, Richard David et al
BP INTERNATIONAL LIMITED Patents
Division Chertsey Road
Sunbury-on-Thames Middlesex TW16 7LN
(GB)

(54) **Hydrogenation process.**

(57)    A process for the production of tertiary alkyl ethers from a feed containing tertiary alkenes and
diolefins comprises the following steps :
    (a) subjecting the feed to a hydrogenation step to selectively hydrogenate the diolefins employing, as
catalyst, sulphided nickel on a support, the catalyst containing at least 40% by weight of nickel based on
the total weight of nickel and support,
    (b) subjecting the hydrogenated feed to an etherification step which comprises reacting the tertiary
alkenes with an alcohol in the presence of an etherification catalyst to form tertiary alkyl ethers.
    The feed containing tertiary alkenes can be a light catalytically cracked spirit obtained from the
catalytic cracking of mineral oils and the alcohol can be methanol and the product from the
etherification step contain one or more of tertiary butyl methyl ether, tertiary amyl methyl ether, tertiary
hexyl methyl ether and tertiary heptyl methyl ether.

Fig. 2

EP 0 490 571 A2

The present invention relates to a process for the production of tertiary alkyl ethers.

Tertiary alkyl ethers for example methyl tertiary butyl ether (MTBE) and tertiary amyl methyl ether (TAME) are valuable additives for increasing the octane number of gasoline. They may be made by the reaction of tertiary alkenes (iso-alkenes which contain a double bond adjacent to a tertiary carbon atom) with an appropriate alcohol, e.g. methanol. Various processes have been published for carrying out this reaction. EP 87 658 discloses the catalytic conversion of iso-alkenes by reaction with an alkanol with simultaneous removal of acetylene, carbonyl compounds and diolefin compounds by hydrogenation. EP 338 309 relates to a process for isomerisation of alkenes. This isomerisation process can be combined with etherification and with hydrogenation of certain highly unsaturated components in the feed mixture.

An article by P M Lange et al in "Hydrocarbon Processing" December 1985 pages 51-52 discusses the etherification of cracked feeds containing diolefins and indicates that such diolefins can cause problems in subsequent processes by deactivating the catalyst. Such diolefins may be removed by hydrogenation reactors

There is no suggestion in this reference that the presence of diolefins is harmful to the etherification catalyst. The fact that it has been proposed to combine etherification with selective diene hydrogenation indeed suggests that the presence of such diolefins in contact with the etherification catalyst can be tolerated. As indicated above the presence of diolefins in the unreacted hydrocarbon product leaving the etherification process may have an adverse effect in some processes to which this unreacted material is subsequently fed. However the logical place for removing any such unwanted diolefin is downstream of the etherification reactor because the bulk of material to be handled will be reduced as a result of the reaction of the tertiary alkenes while there will be no risk of reducing the yield of the desired tertiary alkyl ethers as a result of any hydrogenation of the tertiary alkenes.

European Patent No. 035935 describes a process for the production of tertiary amyl methyl ether from a $C_5$ fraction in which the $C_5$ fraction is hydrogenated before reaction with methanol. The objective of the hydrogenation is to remove not only diolefins but also unreactive monolefins. The stated advantage of the process is to increase the octane number of the resulting product. The recommended catalysts are nickel and palladium which are described in the metallic form. This is consistent with the hydrogenation being a relatively severe one. As a consequence, not only a portion of the olefins which are unreactive with regard to etherification is hydrogenated, but also a considerable portion of those which can be etherified, particularly tertiary alkenes. This portion is lost for the production of octane number increasing ethers.

We have now found that we can improve the catalyst life in the etherification process if the feed is first subjected to a relatively mild hydrogenation step using a sulphided nickel catalyst to selectively hydrogenate the diolefins.

According to the present invention a process for the production of tertiary alkyl ethers from a feed containing tertiary alkenes and diolefins comprises the following steps:

(a) Subjecting the feed to a hydrogenation step to selectively hydrogenate the diolefins employing, as catalyst, sulphided nickel on a support, the catalyst containing at least 40% by weight of nickel based on the total weight of nickel and support,

(b) Subjecting the hydrogenated feed to an etherification step which comprises reacting the tertiary alkenes with an alcohol in the presence of an etherification catalyst to form tertiary alkyl ethers.

Preferred feedstocks are tertiary olefin and diolefin containing hydro-carbon cuts obtained by the catalytic cracking of heavier petroleum fractions normally boiling in the range 350°C to 550°C or by the steam cracking of naphtha. The feedstocks may contain sulphur components up to 10,000 ppm (calculated as sulphur).

The catalysts are preferably relatively high molecular weight solid carbonaceous materials containing $SO_3H$ groups. The most preferred are strongly acidic cation exchange resins consisting essentially of a sulphonated polystyrene resin, for instance a divinylbenzene cross-linked polystyrene matrix attached to which are sulphonic acid groups. These resins may be gel like or macroreticular. They are manufactured and sold commercially under various trade names.

In the etherification the LHSV (liquid hourly space velocity) is suitably 0,1 -10, preferably 0,5 - 3 $h^{-1}$; the reactor temperature is suitably 40-90 °C, preferably 60-71 °C; the pressure is high enough to keep all components in the liquid state, normally 10-20 barg are sufficient; the molar ratio of alcohol to reactive olefins is suitably in the range 0,7 - 3, preferably 0,8-2,0. (The reference is barg is to gauge pressure in bar. To obtain absolute pressure add 1 bar).

The term diolefin in the specification means a molecule containing two carbon carbon double bonds. In most cases the double bonds are conjugated as, for example, in 1,3 butadiene.

The selective hydrogenation of diolefins in the presence of monoalkenes has been previously described in, for example, an article by Anderson et al in Ind. Eng. Chem. December 1948 pages 2295-2301. The use of sulphided nickel catalysts to selectively hydrogenate diolefins is also disclosed in a paper by K H Bourne et al in the Proceedings of the Third International Congress on Catalysis Amsterdam 1964.

Conditions for use in the selective hydrogenation process can be readily determined for any given selective hydrogenation catalyst by those skilled in the art.

Preferably the nickel content of the catalyst for the selective hydrogenation is at least 50%, for example, at least 60% and can be up to 75 weight percent based on the total weight of nickel and support. The preferred support is alumina. In the present invention these catalysts are used in the sulphided form.

Suitable catalysts contain at least 2% by weight of sulphur based on the total weight of nickel and sulphur. Preferably the catalyst is saturated with sulphur, the content of which is then about 3 to 5%, typically about 4% by weight. Saturation is reached when the sulphur content of the catalyst cannot be increased any more. This point can be easily determined by those skilled in the art. Preferably the catalyst is presulphided before use in the hydrogenation stage. Sulphiding may be carried out by contacting the hydrogen activated catalyst with a liquid hydrocarbon containing dimethyl disulphide. Hydrogen sulphide or mercaptans may also be used.

The feed to the hydrogenation step can contain up to 5% by weight of diolefins though even higher contents can be tolerated. The hydrogenation step can be carried out using conditions in the following ranges:

LHSV: Suitably 0.5 to 10, preferably 1 to 6 vol/vol.h

Temperature: Suitably 50 to 120°C, preferably 60 to 100 °C

Pressure: Suitably 10 to 30, preferably 15 to 20 barg

Molar ratio hydrogen/diolefins at least 1:1.

The optimum conditions can be easily determined for any feedstock by those skilled in the art. It is possible to convert the diolefins selectively to an extent of more than 70%, usually more than 80% by weight. However, conversion rates of less than 70% can lead to a considerable improvement in the life of the etherification catalyst.

In one embodiment of the process the feed containing tertiary alkenes and diolefins is a light catalytically cracked spirit having an initial boiling point in the range 25 to 40°C and a final boiling point in the range 100 to 120°C obtained from the catalytic cracking of mineral oils, the alcohol is methanol and the product from the etherification step contains one or more of tertiary butyl, tertiary amyl, tertiary hexyl and tertiary heptyl methyl ether.

The invention will now be described with reference to the drawings in which:

Figure 1 is a diagramatic representation of a process for making tertiary alkyl ethers not in accordance with the invention and,

Figure 2 is a diagramatic representation of a process according to the present invention.

The invention is further illustrated by reference to the following examples in which the example of the invention is identified by number and the comparative test not according to the invention is identified by letter.

## Comparative Test A

A feed consisting of a mixture of light catalytically cracked spirit (LCCS) and methanol was fed to an etherification reactor system through line (1). Samples could be taken off at sample point (2). The mixed feed was fed to reactor (3). This was a guard reactor intended to protect the main reactor from poisoning by basic components and metal cations. The guard reactor contained 500 ml of a standard ion exchange etherification catalyst (in the acid form) commercially available from Bayer AG under the designation K2631. The mixed feed was then passed through line (4) via sample point (5) and a temperature controlled oil bath (6) into the main reactor (7), which also contained 500 ml of ion exchange catalyst K2631. Part of the product from the main reactor (7) was recycled through line (8) while the remainder was taken off past sample point (9) through line (10).

The LCCS was a product derived from the catalytic cracking of mineral oils. The main characteristics of the LCCS are set out below:

ASM Distillation

| | |
|---|---|
| Initial Boiling Point | 32.5 °C |
| 5 % vol recovered by | 40.2 °C |
| 50 % vol recovered by | 54.8 °C |
| 95 % vol recovered by | 96.4 °C |
| Final Boiling Point | 109.4 °C |
| Yield | 96.7 % |
| Density at 15°C | 678.5 kg/m$^3$ |

| Gum (EN 5) | Less than 1 mg/dl |
| Vapour Pressure (EN 12) | 0.86 barg |
| Sulphur Total | 380 mg/kg |
| Nitrogen Total | 6 mg/kg |
| Nitrogen Basic | 1 mg/kg |
| Water | 27 mg/kg |

Hydrocarbon distribution:

| $C_4$ | 5,0 % wt |
| $C_5$ | 43,3 % wt |
| $C_6$ | 40,3 % wt |
| $C_7$ | 11,4 % wt |
| Sum | 100 % wt |

Identified Dienes by HPLC/GC:

| C4 | 0.010 % wt |
| C5 | 0.320 % wt |
| C6 | 0.379 % wt |
| C7 | 0.060 % wt |
| Sum | 0.769 % wt |

In addition to the dienes mentioned above the LCCS contained various reactive isoalkenes capable of reacting with methanol to give tertiary alkyl ethers. Thus it contained isobutene (0.41 %), 2-methyl-butene-1 (3.70 %), 2-methyl butene-2 (7.41 %), 2,3-dimethyl butene-1 (0.44 %), 2-methyl pentene-1 (1.44 %), 2-ethyl butene-1 (0.38 %).

The mixture of LCCS and methanol contained 9.96 % wt methanol (calculated as corresponding to a mole ratio of methanol to reactive olefins of 1.05:1).

The etherification conditions used in reactor (7) were LHSV 2 $h^{-1}$ (11 fresh feed per hour), pressure 10 barg, reactor temperature 60°C. The ratio of fresh feed:recycle volume was 1:1 (recycle volume 11 per hour). LHSV and pressure in the guard reactor (3) were the same as in the main reactor, however, the temperature was maintained at 2°C (means not shown in the flow diagram).

The etherification process was carried out continuously for 94 days. On the 60th day the temperature in the reactor was increased from 60°C to 65°C because the ether yield had fallen steeply.

Samples of material were taken from the reactor system at intervals of 14 days for analysis by gas chromotography. These samples were first taken with the LHSV of 2. The LHSV was then increased to 4 and the reactor system allowed to stabilise. (The LHSV was again calculated on the basis of the fresh feed flow rate; the recycle volume of 11 per hour was, however, not changed). The samples were then taken at the increased LHSV. After the samples had been taken the LHSV was again reduced to 2.

A good indication of deactivation of the etherification catalyst is the change in the TAME yield. The deactivation of the catalyst was already noticeable after 14 days on stream. With increasing time on stream the TAME yield fell more steeply. After 59 days on stream the original yield of 56,5 % (determined on the 3rd day) had fallen to 34.8 % and as indicated above the temperature of the etherification step was increased by 5°C to 65°C. After this the next sample taken after 66 days on stream showed a TAME yield of 39.3 %. However the deactivation effect could not be stopped. After 93 days on stream the TAME yield was only 21.1 %.

Tests carried out at the LHSV of 4 showed the same general picture but the effect of deactivation showed itself even more clearly than with the LHSV of 2. The TAME yield fell from an original 44.0 % on the 3rd day on stream to 12.5 on the 93rd day on stream.

The yield of MTBE did not show such a dramatic change as that for TAME. However a distinct deactivation was noticeable. After 59 days on stream the MTBE yield fell from an original value of over 90 % to 73.6 %. After the temperature increase to 65°C the value of 84.3 % was observed. However after 93 days on stream the MTBE yield showed only a value of 61.7 %.

The deactivation at LHSV 4 was particularly noticeable. The MTBE yield fell here from about 85 % on the 3rd day on stream to 44.6 % after 93 days on stream.

The $C_6$-methyl ether yield fell in the course of the experiment from 29.4 % after 3 days on stream to 9.3 % after 93 days on stream. After temperature increase on the 59th day on stream only a small increase in the ether yield was observed. The deactivation of the catalyst was again more noticeable with LHSV 4 where the yield fell from 22.6 % on the 3rd day on stream to 4.9 % after 93 days on stream.

Example 1

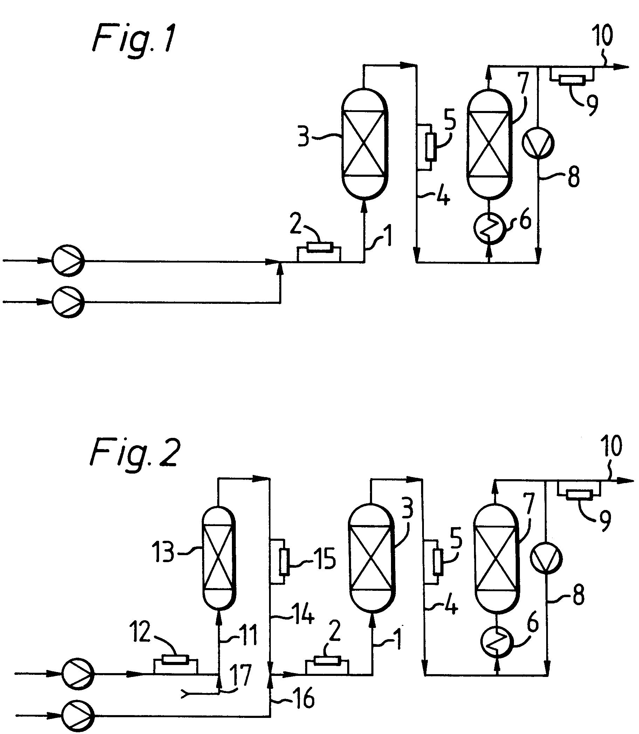

An apparatus was used identical to that used in Comparative Test A except that the LCCS was fed to a hydrogenation reactor before being mixed with methanol and fed to the guard reactor. The apparatus used is shown in Figure 2. LCCS is fed through a line (11) past sample point (12) and into hydrogenation reactor (13). A small amount of hydrogen was fed into the feed in line (11) through line (17). The product from the hydrogenation reactor is fed through line (14) past sample point (15) where it is mixed with methanol fed through line (16) and the mixture fed through line (1) past sample point (2) into guard reactor (3). The other components of the apparatus are the same as in Figure 1.

The catalyst used in the hydrogenation reactor was a nickel containing catalyst commercially available in the designation NI-5124T from Engelhard. This catalyst contained 63 % nickel on alumina. The catalyst was supplied in reduced form and the surface was stabilised by absorption of $CO_2$.

The catalyst was activated in situ in the hydrogenation reactor which had a capacity of 350 ml. The reactor was filled with catalyst. n-Heptane was pumped through the catalyst with an LHSV of 1 $h^{-1}$ at a pressure of 50 barg. A hydrogen stream of 5 litres/hour (measured at normal conditions) was fed through the reactor, which was slowly heated to 100°C. A reaction front moved through the reactor. This could be checked by observation of the reactor temperature. After the reaction had passed through the reactor the reactor was heated to 150°C and held at these conditions for 24 hours.

After this step the catalyst was activated.

It was now necessary to sulphide the catalyst to reduce its activity so that desirable reactive monoalkenes would not be hydrogenated. n-Heptane was again pumped through the reactor with an LHSV of 2 and a pressure of 15 barg. A hydrogen stream was fed through the reactor at the rate of 6 litres/hour (normal conditions) while maintaining a temperature of 80°C. While maintaining these conditions an n-heptane solution containing 1.5 % dimethyl disulphide was fed through the reactor. This solution was pumped through the reactor for 5 hours.

The reactor system with the selective hydrogenation step was operated in parallel with the reactor system of Comparative Test A starting with the same LCCS feed and with the same etherification conditions. The selective hydrogenation step was operated with an LHSV of 2.6 $h^{-1}$ with a pressure of 15 barg and a reaction temperature of 80°C at a hydrogen flow rate of 8 litres/hour (normal conditions).

The dienes were substantially removed by the hydrogenation step. Hydrogenation of the reactive isoalkenes was not observed

In addition to the diene removal double bond isomerisation was observed. Both desirable and undesirable isomerisations are possible. In desirable isomerisations an isoalkene which is not a tertiary alkene is isomerised to a tertiary alkene. In undesirable isomerisations a reactive isoalkene-1 is isomerised to an isoalkene-2 which etherifies less readily. However isomerisation was found to give a positive effect overall as the TAME yield increased by 2.5 % absolute compared with the material which had not been subjected to selective hydrogenation.

The results of the experiment showed that the pre-hydrogenated LCCS produced more TAME from the beginning than was the case with the untreated LCCS. When on the 59th day the temperature in the parallel operated etherification reactors was increased to 65°C because the TAME yield in the Comparative Test A unit had already fallen to 34.8 % the TAME yield in the unit operated according to the invention was still 46.6 %. After the increase in temperature a TAME yield of 59.1 % was measured. At the end the TAME yield was still 46.2 %. This was more than twice the TAME yield for Comparative Test A.

The MTBE yield in the unit operated according to the invention fell only slightly during the 94 days on stream.

The yield of $C_6$ methyl ethers was higher than that found with Comparative Test A. With LHSV of 2 the yield on the 4th day on stream was 30.7 % and on the 94th day it was 21.0 %. At the increased LHSV of 4 the yield on the 4th day was 24.9 % and on the 94th day it was 15.1 %.

Example 2

This example demonstrates the benefit of using in the hydrogenation step a catalyst containing more than 40% by weight of nickel in accordance with the invention.

The results are summarised in the Table.

5

| Operating Temperature | Conversion obtained with catalyst containing | | |
|---|---|---|---|
| | 11% Nickel | 60% Nickel | 63% Nickel |
| 50°C | 5% | 17% | 57% |
| 65°C | 6% | 50% | 75% |
| 80°C | 8% | 70% | 88% |

The LHSV in every case was 3 vol/vol/hour. Pressure was 15 bar gauge.

The 11% nickel catalyst was a Engelhard NI-0301T catalyst.

The 60% nickel catalyst was a Engelhard NI-3288 catalyst.

The 63% nickel catalyst was a Engelhard NI-5124T catalyst.

All three catalysts were sulphided in the same way in the manner described in Example 1.

The conversions recorded are for the sum of $C_4$ to $C_7$ diolefins.

These results show that by operating with a catalyst according to the invention a much higher conversion of diolefins is obtained. Moreover the hydrogenation is very selective. The concentration of reactive mono olefins is not only not decreased, but even increased by isomerisation of non-reactive mono olefins.

## Claims

1. A process for the production of tertiary alkyl ethers from a feed containing tertiary alkenes and diolefins which process comprises the following steps:

    (a) subjecting the feed to a hydrogenation step to selectively hydrogenate the diolefins employing, as catalyst, sulphided nickel on a support, the catalyst containing at least 40% by weight of nickel based on the total weight of nickel and support.

    (b) subjecting the hydrogenated feed to an etherification step which comprises reacting the tertiary alkenes with an alcohol in the presence of an etherification catalyst to form tertiary alkyl ethers.

2. A process as claimed in Claim 1 wherein the catalyst in the hydrogenation step contains at least 50% by weight of nickel.

3. A process as claimed in Claim 1 or 2 wherein the catalyst employed in the hydrogenation step contains at least 2% by weight of sulphur based on the total weight of nickel and sulphur.

4. A process as claimed in any one of Claims 1 to 3 wherein the hydrogenation step comprises contacting the feed with hydrogen at a temperature of 50 to 120°C
    LHSV of feed        0.5 to 10 vol/vol/hour
    and pressure        10 to 30 bar gauge.

5. A process as claimed in Claim 4 wherein the molar ratio of hydrogen to diolefins at least 1:1.

6. A process as claimed in any one of the preceding Claims wherein the etherification step is carried out under the following conditions:
    LHSV from 0.1 to 10 vol/vol/hour
    temperature from 40 to 90°C
    molar ratio of alcohol to tertiary alkenes from 0.7 to 1 to 3.0 to 1 and the
    pressure is sufficient to maintain all components in the liquid phase.

7. A process as claimed in any one of the preceding Claims wherein the feed containing tertiary alkenes and diolefins is a light catalytically cracked spirit having an initial boiling point in the range 25 to 40°C and a final boiling point in the range 100 to 120°C obtained from the catalytic cracking of mineral oils, the alcohol is methanol and the product from the etherification step contains one or more of tertiary butyl methyl ether, tertiary amyl methyl ether, tertiary hexyl methyl ether and tertiary heptyl methyl ether.

*Fig.1*

*Fig.2*